# EUROPÄISCHE PATENTSCHRIFT

(11) **EP 3 061 116 B1**
(45) Veröffentlichungstag und Bekanntmachung des Hinweises auf die Patenterteilung: **01.08.2018**
(21) Anmeldenummer: 14792390.8
(22) Anmeldetag: 21.10.2014
(51) Int. Cl.: A61B 6/00, H01J 35/02, H01J 35/06, H01J 35/08

(54) **TARGET UND/ODER FILAMENT FÜR EINE RÖNTGENRÖHRE, RÖNTGENRÖHRE, VERFAHREN ZUR ERKENNUNG EINES TARGETS UND/ODER EINES FILAMENTS UND VERFAHREN ZUR EINSTELLUNG DER KENNGRÖSSEN EINES TARGETS UND/ODER EINES FILAMENTS**
TARGET AND/OR FILAMENT FOR AN X-RAY TUBE, X-RAY TUBE, METHOD FOR IDENTIFYING A TARGET AND/OR A FILAMENT AND METHOD FOR SETTING THE CHARACTERISTICS OF A TARGET AND/OR A FILAMENT
CIBLE OU FILAMENT POUR TUBE À RAYONS X, TUBE À RAYONS X, PROCÉDÉ DE DÉTECTION D'UNE CIBLE ET/OU D'UN FILAMENT ET PROCÉDÉ DE RÉGLAGE DES CARACTÉRISTIQUES D'UNE CIBLE ET/OU D'UN FILAMENT

(30) Priorität: 21.10.2013 DE 102013017463
(43) Veröffentlichungstag der Anmeldung: 31.08.2016
(73) Patentinhaber: YXLON International GmbH, 22419 Hamburg (DE)
(72) Erfinder: NIEMANN, Willhelm, 22846 Norderstedt (DE); SCHRADER, Björn, 33765 Hamburg (DE); DEYE, Jens, 20255 Hamburg (DE); KLEIN, Axel, 22175 Hamburg (DE)
(74) Vertreter: DTS Patent- und Rechtsanwälte Schnekenbühl und Partner mbB
(86) Internationale Anmeldenummer: PCT/EP2014/002839
(87) Internationale Veröffentlichungsnummer: WO 2015/058853

(56) Entgegenhaltungen:
- WO-A1-00/58991
- WO-A2-2004/023852
- DE-A1- 10 146 210
- JP-A- 2003 115 398

## Beschreibung

Die Erfindung befasst sich mit einem Target und/oder einem Filament für eine Röntgenröhre, mit einer Röntgenröhre, die ein solches Target und/oder ein solches Filament aufweist, mit einem Verfahren zur Erkennung eines solchen Targets und/oder eines solchen Filaments und mit einem Verfahren zur Einstellung der Kenngrößen eines solchen Targets und/oder eines solchen Filaments

Aus dem Stand der Technik sind Röntgenstrahlenquellen bekannt, die man grob in zwei Gruppen - geschlossene und offene Röhren - unterteilen kann.

Die geschlossenen Röhren sind ab Produktion in ihren Parametern und Eigenschaften festgelegt, d.h. das Vakuum wird bei der Produktion erzeugt und es gibt keine Möglichkeit nach der Produktion Änderungen an der Röhre durchzuführen.

Anders verhält es sich bei den offenen Röhren. Hier wird das Vakuum der Röhre erst bei der Inbetriebsetzung erzeugt und kann auch im Bedarfsfalle (öffnen der Röhre) wieder abgebaut werden. Damit haben die offenen Röhren mehrere entscheidende Vorteile. Zum einen können defekte Teile ausgetauscht werden und was viel bedeutender ist, es können durch den Austausch von Komponenten die Eigenschaften der Röhren verändert werden.

Gerade im Bereich der hochauflösenden Röntgenröhren kann es zum Beispiel notwendig sein je nach Anwendungsfall Brennfleckgröße oder Leistung zu verändern. Dies kann durch den Einsatz eines geeigneten Targets erfolgen.

Der Bediener hat somit den Freiheitsgrad, aus verschiedenen Targets das für seine Applikation/Prüfaufgabe geignete Target auszuwählen.

Für jedes dieser Targets gibt es in der Röhrensteuerung einen passenden Parametersatz - im Folgenden als Kenngrößen bezeichnet -, der den optimalen und sicheren Betrieb der Röntgenröhre mit dem Target gewährleistet. Ein falscher Parametersatz führt unweigerlich zu schlechten Prüfergebnissen bis hin zur Zerstörung des Targets.

Stand heute ist es erforderlich, dass der Bediener die notwendigen Parameter an der Röhrensteuerung passend zum Target auswählt und einstellt. Dieser manuelle Eingriff in das System ergibt sich daraus, dass es aufgrund der Einbausituation in der Röntgenröhre keine Möglichkeit gibt, das Target zu erkennen.

Röntgenröhren sind entweder als Transmissionsstrahler - bei diesen trifft der Elektronenstrahl senkrecht auf das Target und die Röntgenstrahlung wird durch das Target emittiert - oder als Direktstrahler - bei diesen trifft der Elektronenstrahl unter einem Winkel auf das Target und die Röntgenstrahlung tritt aus der gleichen Fläche mit entsprechendem Austrittswinkel aus - ausgebildet.

Die weiteren Darstellungen und Betrachtungen werden am Beispiel eines Transmissionsstrahlers dargestellt; sie sind aber analog auf einen Direktstrahler übertragbar und gelten somit für alle offene Röhren. Für geschlossene Röhren ist dies - für den Fachmann erkennbar - prinzipiell auch nicht anders zu betrachten, so dass die Ausführungen im Wesentlichen auch auf diese übertagbar sind.

Die übergeordnete Aufgabe ist es, eine Eliminierung möglicher Fehlbedienungen und damit eine signifikante Steigerung der Betriebssicherheit und damit auch der Lebensdauer einer Röntgenröhre zu erhalten. In der Folge ergeben sich dadurch als weitere Teilaufgaben auch eine Steigerung der Prozesssicherheit bei Maschinen, in denen solche Röntgenröhren zum Einsatz kommen.
Die Aufgabe wird durch ein Target mit den Merkmalen des Patentanspruchs 1 gelöst. Aufgrund des Identifizierungselements kann durch das Zusammenwirken mit des Erfassungselement eine Lösung gefunden werden, welche insbesondere bei einer offenen Röhre beim Targetwechsel die Möglichkeit bereitstellt, das entsprechende Target automatisch zu ermitteln. Damit wird dann die angeschlossene Steuerung in die Lage versetzt die zugehörigen Kenngrößen mit den dazu gehörigen Betriebsparametern bereitzustellen. Die erhaltene Information kann in einer angeschlossenen Steuerung beziehungsweisen einem PC ausgewertet und der notwendige Parametersatz und damit die möglichen Einstellungen aktiviert und bereitgestellt werden. Damit ist eine Fehlbedienung durch den Bediener eliminiert. Weitergehend wird damit die Basis geschaffen, eine vorrausschauende Bedienerführung zu etablieren, wodurch typische Betriebsparameter des Targets nun auch als Betriebsdaten hinterlegt und damit auch überwacht werden können, ohne aufwendige und fehleranfällige Bedienereingriffe.

Die Aufgabe wird auch durch ein Filament mit den Merkmalen des Patentanspruchs 2 gelöst. Das zuvor hinsichtlich Patentanspruch 1 für ein Target Gesagte gilt analog auch für das Filament.

Eine vorteilhafte Weiterbildung der Erfindung sieht vor, dass das Identifizierungselement ein elektrisches Identifizierungselement, ein optisches Identifizierungselement oder ein mechanische Identifizierungselement ist.

Eine weitere vorteilhafte Weiterbildung der Erfindung sieht vor, dass das elektrische Identifizierungselement eine elektronische Komponente, insbesondere ein RFID-Chip, ist und/oder das optische Identifizierungselement ein Strichcode oder ein QR-Code ist und/oder das mechanische Identifizierungselement Durchbohrungen, Wellenschliffe oder Kerben aufweist.

Die Aufgabe wird auch durch eine Röntgenröhre mit den Merkmalen des Patentanspruchs 5 gelöst. Dies geschieht dadurch, dass das Identifizierungselement des Targets und/oder des Filaments mit dem Erfassungselement zusammenwirkt.

Eine vorteilhafte Weiterbildung der Erfindung sieht vor, dass das Erfassungselement eine elektronische Abtastvorrichtung, eine optische Kamera, eine Mechanik oder eine Sensorik ist. Dadurch kann neben der Target- beziehungsweise der Filämenterkennung auch eine Seriennummer des Targets/Filaments erfasst werden. Damit ergibt sich eine eindeutige Zuordnung und die Möglichkeit die Lebensdauer bzw. die Betriebsdauer eines Targets/Filaments trotz Tragetbeziehungsweise Filamentwechsels ohne einen Bedienereingriff mit zu loggen. Dies ist insbesondere dann von Nutzen, wenn der Bediener eine Prüfaufgabe starten möchte, deren Durchführungszeit erheblich länger ist als die verbleibende Lebenszeit des Targets.

Die Aufgabe wird auch durch ein Verfahren mit den Merkmalen des Patentanspruchs 7 gelöst. Durch das Zusammenwirken von Identifizierungselement an der Targetbasis und Erfassungselement wird nach Abgleich der erkannten Merkmale des Identifizierungselenents über eine Datenbank eindeutig das Target beziehungsweise das Filament erkannt. Daraus ergeben sich die oben schon genannten Vorteile.

Alternativ wird die Aufgabe auch durch ein Verfahren mit den Merkmalen des Patentanspruchs 8 gelöst. Dadurch ist kein Indentifizierungselement am Target nötig.

Alternativ wird die Aufgabe auch durch ein Verfahren mit den Merkmalen des Patentanspruchs 9 gelöst. Auch hier ist kein Indentifizierungselement am Target nötig. Hierbei erfolgt die Identifikation dadurch, dass beim Start der Röntgenröhre zuerst eine immer gleiche vorgegebene Betriebsart angefahren wird, bei der bei gleichen Ansteuerparametern jedes Target eine unterschiedliche Röntgenstrahlung emittiert, und die dabei entstehende Röntgenstrahlung ausgewertet wird. Somit wird ohne jegliches Zusatzelement bei nur kurzer Prüfzeit die Indentifizierung des Targets erreicht.

Die Aufgabe wird auch durch ein Verfahren mit den Merkmalen des Patentanspruchs 10 gelöst. Hierbei wird nicht nur das Target/Filament erkannt, sondern auch die für dieses konkrete Target/Filment nötigen Kenngrößen eingestellt.

Weitere Vorteile und Einzelheiten der Erfindung werden anhand der in den Figuren dargestellten Ausführungsbeispiele erläutert. Die Figuren zeigen:
- Figur 1: eine schematische Darstellung einer bekannten Röntgenröhre,
- Figur 2: eine Ansicht eines erfindungsgemäßen Targets mit elektrischem Identifizierungselement,
- Figur 3: das Target der Figur 2 im eingebauten Zustand,
- Figur 4: schematische Darstellung eines Verfahrens mittels Widerstandsmessung,
- Figur 5: Darstellung eines erfindungsgemäßen rotierenden Targets mit Barcode,
- Figur 6: Darstellung eines feststehenden Targets mit Barcode im nichteingebauten Zustand,
- Figur 6a: Darstellung des Targets der Figur 6 im eingebauten Zustand,
- Figur 7: Darstellung eines erfindungsgemäßen Targets mit mechanischem Identifizierungselement im nicht eingebauten Zustand,
- Figur 7a: Darstellung des Targets der Figur 7 im eingebauten Zustand und
- Figur 8: schematische Darstellung eines konventionellen Targets und eines Targets zur Durchführung einer Erkennung mittels "Defokussierung".

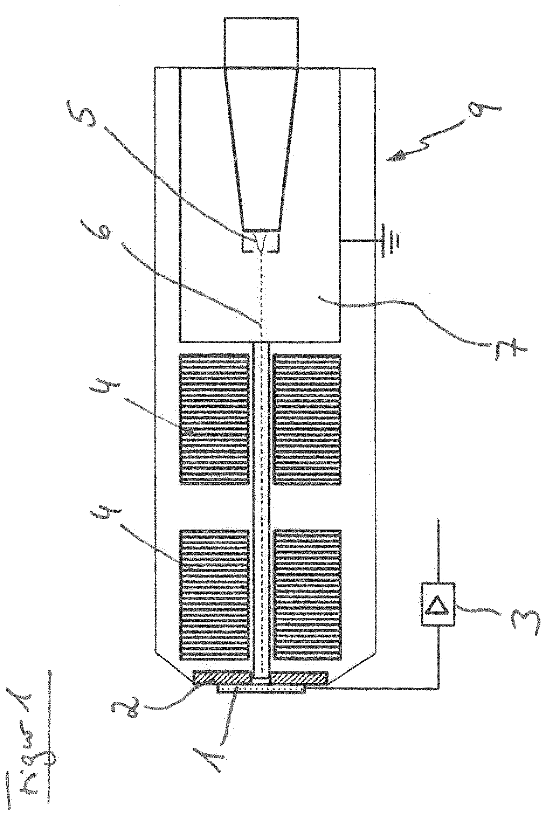

Die Target-Erkennung ist problematisch. Anhand von Figur 1 wird schematisch der Aufbau einer Röntgenröhre 9 erläutert. Das Target 1 befindet sich an einem exponierten Ort des, in weiten Teilen aus massiven Eisen bestehenden Kopfes der Röntgenröhre 9. Dieser Einbauort besticht durch seine hohe Betriebstemperatur, hohe Strahlendosis sowie den magnetischen Feldern der Fokusspule. Zur Regelung und Steuerung des Röntgenstrahls ist es erforderlich, den auf das Target 1 - genauer: auf das Targetelement 11, 11a, 11b - auftreffenden Elektronenstrahl 6 zu messen. Dies wird durch eine Targetstrommessvorrichtung 3 realisiert. Diese Messung erfordert eine hohe Messgenauigkeit im Bereich von 1-3000 µA. Für diese Messung ist es notwendig, das Targetelement 11, 11a, 11b elektrisch isoliert von der Röntgenröhre 9 über einen elektrischen Isolator 2 einzubauen. Das Target 1 bei einem Transmissionsstrahler bildet gleichzeitig auch den Abschluss des Vakuums 7 der Röntgenröhre 9. Das Target 1 muss auch bewegt werden können, da nach einer gewissen Nutzungsdauer das strahlungserzeugende Material (z.B. Wolfram) des Targetelements 11, 11a, 11b "verbraucht" oder eingebrannt ist. Dazu dreht man das Target 1 so, dass der Elektronenstrahl 6 auf eine unverbrauchte Position des Targetelements 11, 11a, 11b trifft. Auch sollte der Austausch des Targets 1 sehr einfach sein, um entsprechend den Anforderungen der Prüfaufgaben, einen schnellen Targetwechsel gewährleisten zu können. Äußere Magnetfelder müssen vermieden werden, um die Beeinflussung des Elektronenstrahles 6 (auch der diesen bündelnden magnetischen Linsen 4) vor dem Auftreffen auf das Target 1 zu verhindern. Diese Art der offenen Röntgenröhren 9 wird zum großen Teil für Prüfaufgaben verwendet, bei denen es um sehr kleine Details geht und es auf hohe Vergrößerung im Röntgenbild ankommt. Um das zu realisieren, muss das Prüfobjekt sehr dicht vor dem Target 1 positioniert werden. Für die Konstruktion einer zusätzlichen Targeterkennung ist es also wichtig, dass keine Bauteile in den "Prüfraum" hinein ragen.

Die folgenden Lösungsansätze dienen dazu, das Target 1 an der Röntgenröhre 9 zu identifizieren. Hierbei werden die verwendeten Technologien/Techniken in zwei Gruppen unterteilt:
- Kontakt: es wird eine Verbindung zwischen dem Target 1 und der Technik zur Identifikation hergestellt;
- Berührungslos: es werden Verfahren verwendet, die ohne eine mechanische Verbindung eine Auswertung vornehmen können (z.B.: optische Abtastung, Funk-Abtastung - RFID; Magnetfeld, kapazitiv).

Zum Einsatz kommen unterschiedliche Verfahren.

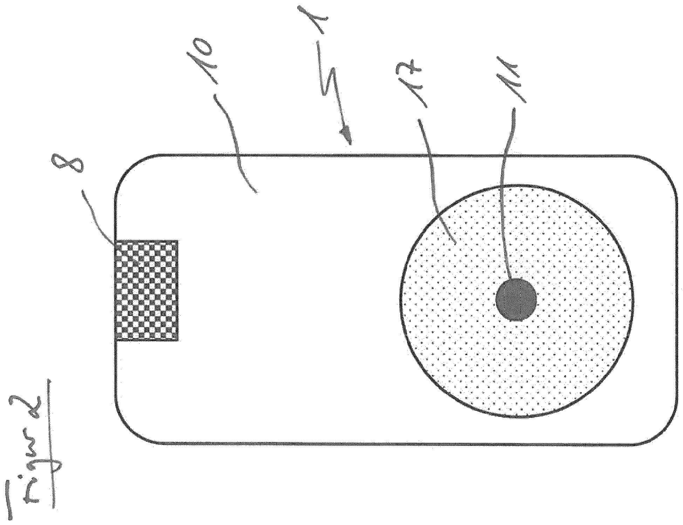

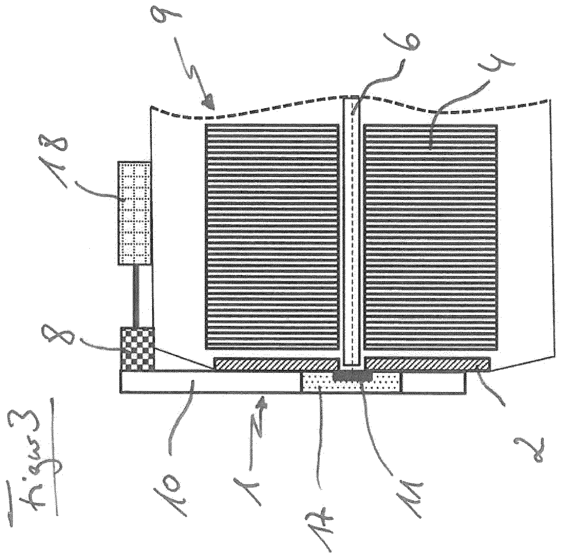

Anhand der Figuren 2 und 3 wird die elektrische Abtastung erläutert. Hier bieten sich zwei Lösungsansätze an. Beispielsweise werden elektronische Komponenten 8 am Target 1 angebracht, die über eine Abtastvorrichtung, ein elektrisches Erfassungselement 18, ausgewertet werden. Als elektronische Komponenten 8 dienen z.B. Widerstände oder eine komplexe elektrische Schaltung. Im ausgebauten Zustand sind diese spannungslos. Durch den Einbau des Targets 1 wird die elektronische Komponente 8 beispielsweise mittels Stecker oder Schleifkontakte mit dem elektrischen Erfassungselement 18 verbunden. Das elektrische Erfassungselement 18 versorgt die elektronische Komponente 8 mit Spannung und wertet die resultierenden Signale aus. Die ausgewerteten Signale überträgt das elektrische Erfassungselement 18 an eine übergeordnete Steuerung, welche dann die zugeordneten Parameter für das Target 1 einstellt.

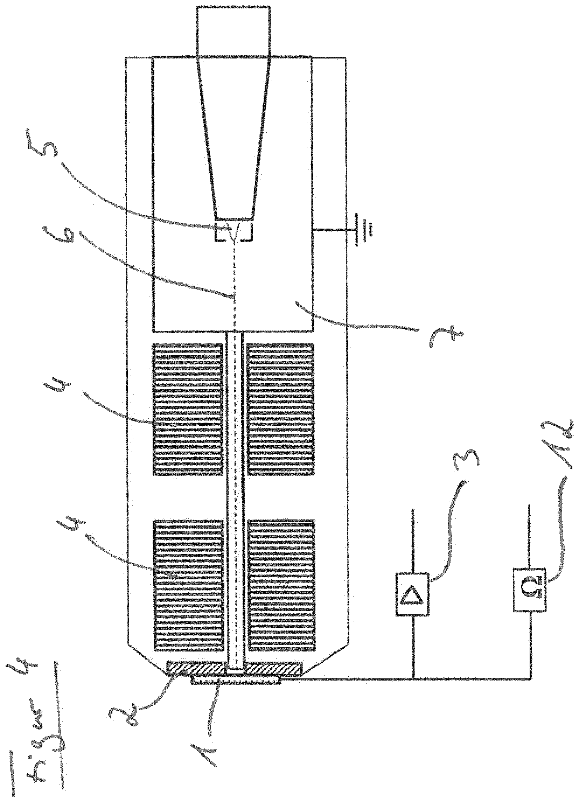

Alternativ zu dem Verfahren gemäß den Figuren 2 und 3 kann bei einer elektrischen Erkennung das Target 1 aufgrund der unterschiedlichen Herstellung selbst in seinem elektrischen Verhalten (z.B. durch Messung der Leitfähigkeit) so verschieden sein, dass eine Identifikation ermöglicht wird. Dies wird anhand Figur 4 erläutert.

Grundlage dabei ist, dass die Materialzusammensetzung der Targets 1 je nach Ausführung unterschiedlich ist und dies durch eine elektrisches Messverfahren (z.B eine Widerstandsmessung mit geeigneter Messspannung mittels einer Targetwiderstandsmessvorrichtung 12) ermittelt werden kann. Um dies zu realisieren, kann die bereits eingebaute Targetstrommessvorrichtung 3 erweitert werden. Wie aus Figur 4 ersichtlich, wurden die aus Figur 1 bekannten Anschlüsse für die Targetstrommessung um eine Targetwiderstandsmessvorrichtung 12 erweitert.

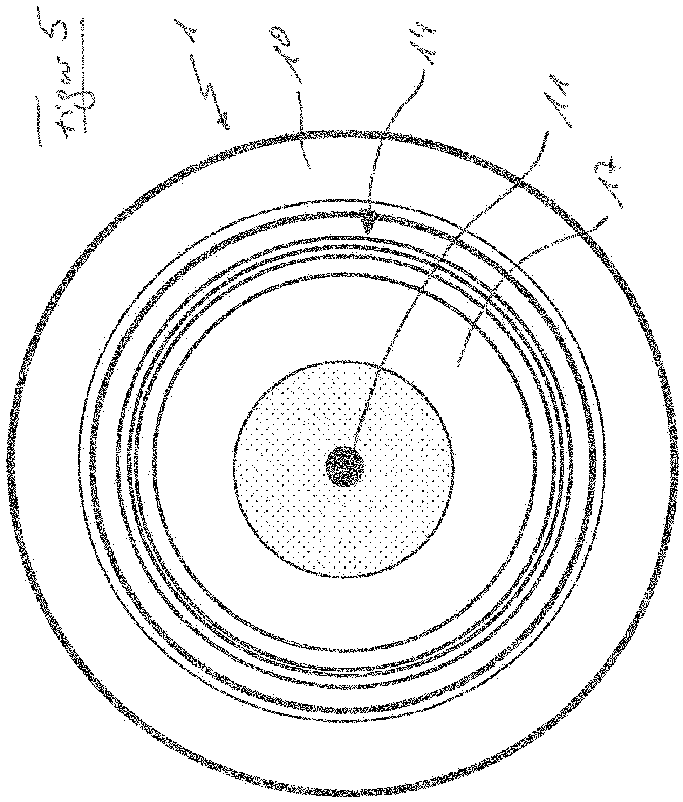

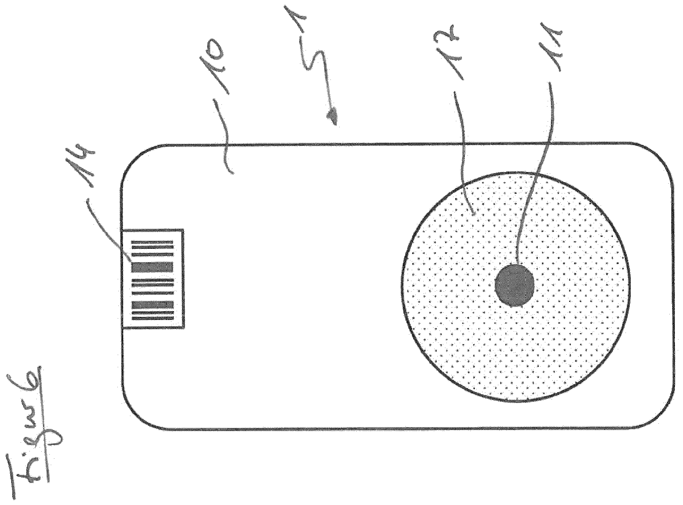

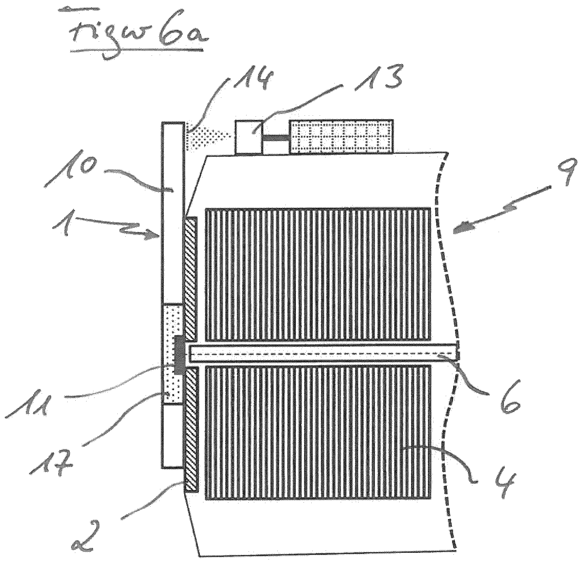

Alternativ zur elektrischen Erkennung kann auch eine optische Erkennung mittels optischer Abtastung durchgeführt werden, wie sie anhand der Figuren 5, 6 und 6a beschrieben wird.

Hier werden an der Targetbasis 10 des Targets 1 optische Marker angebracht, welche durch eine zusätzliche Auswerteeinheit ermittelt werden; in den dargestellten Ausführungsbeispielen handelt es sich um optische Identifizierungselemente 14 jeweils in der Form eines Strich-Codes. Hier kann eine komplexe Abfrage des Strich-Codes oder auch alternativ eines QR-Barcodes durchgeführt werden, welche dann sogar die Seriennummer der Targets 1 enthalten kann. In Figur 5 ist ein rotierendes Target 1 dargestellt, bei der der gesamte Strich-Code konzentrisch um das Drehzentrum des Targets 1 angeordnet ist.

In den Figur 6 und 6a ist ein feststehendes Target 1 dargestellt, dessen optisches Identifizierungselement (Strich-Code 14) - wie bei dem vergleichbaren Ausführungsbeispiel der Figuren 2 und 3 - am oberen Ende der Targetbasis 10 angeordnet ist. Im in Figur 6a dargestellten Einbauzustand des Targets 1 liegt der Strich-Code 14 einem an der Röntgenröhre 9 angebrachten optischen Erfassungselement 13 gegenüber. Als optische Erfassungselemente 13 sind typische Barcodescanner, Kameras aber auch einfache Lichtsensoren, welche Hell-Dunkel-Unterschiede detektieren, einsetzbar. Dadurch kann das optische Erfassungselement 13 die vom Strich-Code 14 herrührenden Signale auswerten. Die ausgewerteten Signale überträgt das optische Erfassungsetement 13 an eine übergeordnete Steuerung, welche dann die zugeordneten Parameter für das Target 1 einstellt.

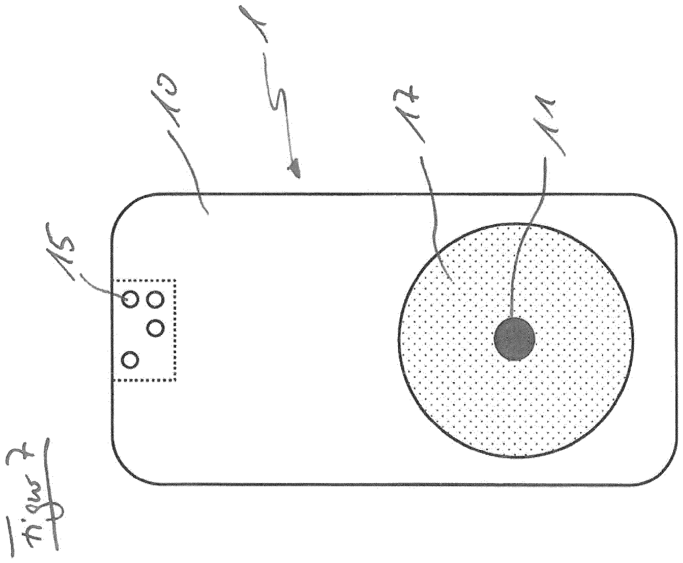

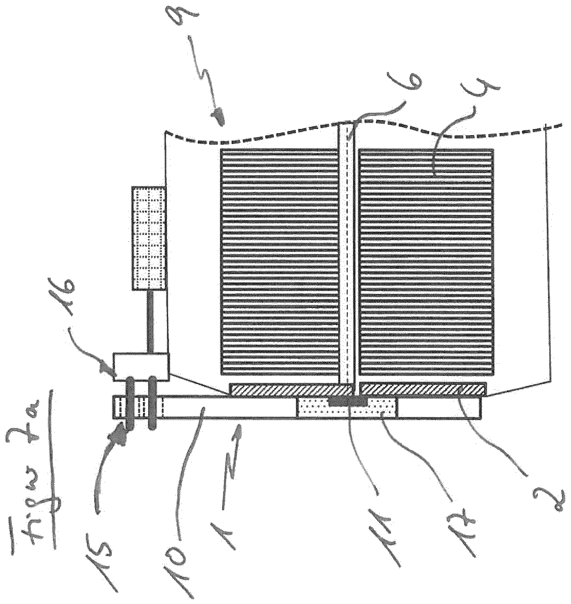

Alternativ zur elektrischen oder optischen Erkennung kann auch eine mechanische Erkennung, beispielsweise anhand der in Figuren 7 und 7a dargestellten Mittel, durchgeführt werden. Das folgende Beispiel zeigt einen möglichen Lösungsansatz bei dem signifikante Durchbohrungen als mechanische Identifizierungselemente 15 an der Targetbasis 10 durch eine Mechanik/Sensorik-Kombination abgefragt werden. Dies kann beispielsweise dadurch erfolgen, dass das mechanische Erfassungselement 16 in Längsrichtung verschiebbare Stifte aufweist, die in die Durchbohrungen 15 am oberen Ende der Targetbasis 10 eintauchen können und somit erfasst wird, ob an der dem jeweiligen Stift zugeordneten Position eine Durchbohrung 15 vorhanden ist oder nicht. Die ausgewerteten Signale überträgt das mechanische Erfassungselement 16 an eine übergeordnete Steuerung, welche dann die zugeordneten Parameter für das Target 1 einstellt.

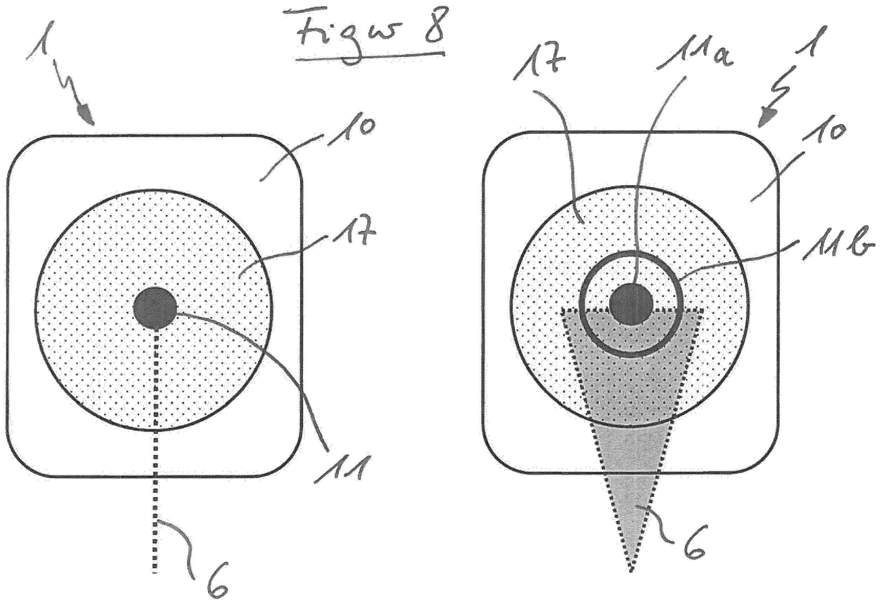

Eine alternative Targeterkennung ohne ein Identifizierungselement an der Targetbasis 10 zu verwenden, wie es beispielsweise auch in der in Figur 4 dargestellt wird (dort als elektrische Erkennung) wird anhand Figur 8 erläutert. Hierbei wird die aus dem Target 1 entstehende charakteristische Strahlung zur Identifikation herangezogen. Dabei sind verschiedene Methoden denkbar:
Bei dieser Lösung wird die Möglichkeit geschaffen, die charakteristische Strahlungseigenschaft des Targets 1 in der Bildkette auszuwerten. Dabei gibt es zwei Lösungsansätze:
- Target 1 bleibt unverändert (siehe linken Teil der Figur 8): die Röntgenröhre 9 wird in einer zur Auswertung definierten Betriebsart betrieben und das daraus resultierende Bild wird ausgewertet. Jedes Target 1 erzeugt durch Einstellung der Betriebswerte Spannung [kV] und Strom [A] eine charakteristische Strahlung. Die Strahlung kann durch die in einer Röntgenanlage eingebaute Bildkette (Detektor inklusive Auswerteeinheit) ausgewertet werden. Um dies zu realisieren, wird eine Betriebsart definiert, bei der alle Targets 1 eines Röhrentyps betrieben werden können. Bei jedem Start der Röntgenröhre 9 wird diese Betriebsart zuerst angefahren, um dann die entstandene Strahlung auszuwerten. Grundlage dieses Verfahren ist, dass bei gleichen Ansteuerparametern jedes Target 1 eine unterschiedliche Strahlung emittiert.
- Target 1 wird modifiziert (siehe rechten Teil der Figur 8): hier wird das Target 1 in der Weise modifiziert wird, damit man durch gezielte "Defokussierung", d.h. Vergrößerung der Elektronenstrahlfläche, auch auf die Außenfläche trifft und dort Strahlung erzeugt. Die daraus signifikant erkennbare Brennfleckänderung ist spezifisch für das Target 1 und kann ausgewertet werden. Im dargestellten Fall (rechte Hälfte - die linke Hälfte stellt ein konventionelles Target 1 dar) ist zusätzlich zu dem kreisförmigen zentralen Targetelement 11a ein durch ein Trägermaterial 17 beabstandetes ringförmiges Targetelement 11b vorhanden. Daran schließt sich eine Bildgebung an, welche mit einem fokussierten Elektronenstrahl 6 abgeglichen ist, d.h. die Bildgebung liefert ein Bild, welches einen gleichförmigen Grauwert zum Inhalt hat. Beim defokussierten Elektronenstrahl 6 ist im vorliegenden Beispiel eine Ringstruktur erkennbar. Andere Formen als die Ringform des ringförmigen Targetelements 11b sind denkbar.

Eine magnetische Auswertung wird nicht anhand von Figuren dargestellt. Es sei nur kurz erläutert, wie diese erfolgt: Es werden Hallsonden an der Röntgenröhre 9 angebracht, um so die Veränderungen des Magnetfeldes resultierend durch ein unterschiedliches Target 1 auszuwerten.

Erfindungsgemäß kann auch ein Multi-Target zum Einsatz kommen, bei dem auf dem Trägermaterial 17 unterschiedliche Targetelemente 11, 11a, 11b (Strahlerzeuger, wie beispielsweise Wolfram) oder unterschiedliche Schichtdicken eines Targetelementes 11, 11a, 11b aufgebracht sind. Durch eine automatische Positionierung können nun die verschiedenen Targetbereiche im Elektronenstrahl 6 positioniert werden.

Die oben erläuterten Lösungsansätze können auf das Filament 5 erweitert werden. Ähnlich wie das Target 1 ist auch das Filament 5 entscheidend für die Bildqualität und auch in unterschiedlichen Ausprägungen vorhanden. Die Funktionsprinzipien der oben beschriebenen Targeterkennung gelten auch hier, sind aber in einem deutlich schwierigeren Umfeld zu realisieren (komplett im Vakuum 7). Dennoch ist eine analoge Anwendung möglich.

Das für die Targeterkennung geschaffene Umfeld an Auswerteelektronik und Steuerung kann relativ einfach erweitert werden, um zusätzliche Betriebsdaten oder Verhalten der Röntgenröhre 9 zu erfassen. Hierzu zählen beispielsweise Biegung der Röntgenröhre 9 bei Temperaturanstieg über zusätzlich angebrachte Dehnungsmesstreifen, allgemeine Temperaturdaten oder eine Magnetfeldanalyse.

Eine derartige Targeterkennung ist auch bei geschlossen Röntgenröhren 9 sinnvoll und die obigen Ausführungen gelten überwiegend auch für diese. Zwar kann man hier kein Target 1 wechseln, allerdings kann man über die Systematik der Targeterkennung auch die für die Röntgenröhre 9 spezifischen Parameter bereitstellen. Die Erkennung kann sich auf den Typ beschränken oder auch das individuelle Target 1 ausgestattet mit Seriennummer betreffen.

### Bezugszeichenliste

- 1: Target
- 2: elektrischer Isolator
- 3: Targetstrommessvorrichtung
- 4: magnetische Linse
- 5: Filament
- 6: Elektronenstrahl
- 7: Vakuum
- 8: elektrisches Identifizierungselement, elektronische Komponente
- 9: Röntgenröhre
- 10: Targetbasis
- 11: Targetelement
- 11a: zentrales Targetelement
- 11b: ringförmiges Targetelement
- 12: Targetwiderstandsmessvorrichtung
- 13: optisches Erfassungselement
- 14: optisches Identifizierungselement, Strich-Code
- 15: mechanisches Identifizierungselement, Durchbohrung
- 16: mechanisches Erfassungselement
- 17: Trägermaterial
- 18: elektrisches Erfassungselement

## Patentansprüche

1. Target (1) für eine Röntgenröhre (9) mit einer Targetbasis (10) und einem daran befestigten Targetelement (11), wobei an der Targetbasis (10) ein Identifizierungselement (8, 14, 15) angebracht ist, das im Zusammenwirken mit einem Erfassungselement (13, 16, 18) an der Röntgenröhre (9) erkennbar ist und das eine eindeutige Zuordnung zu einem passenden Parametersatz in der Röhrensteuerung des Targets (1) aufweist.

2. Filament (5) für eine Röntgenröhre (9) mit einem Filamenthalter und einem daran befestigten Filamentelement, wobei am Filamenthalter ein Identifizierungselement angebracht ist, das im Zusammenwirken mit einem Erfassungselement an der Röntgenröhre (9) erkennbar ist und das eine eindeutige Zuordnung zu einem passenden Parametersatz in der Röhrensteuerung des Filaments (5) aufweist.

3. Target (1) oder Filament (5) nach einem der vorstehenden Patentansprüche, wobei das Identifizierungselement (8, 14, 15) ein elektrisches Identifizierungselement (8), ein optisches Identifizierungselement (14) oder ein mechanische Identifizierungselement (15) ist.

4. Target (1) oder Filament (5) nach Patentanspruch 3, wobei das elektrische Identifizierungselement (8) eine elektronische Komponente (8), insbesondere ein RFID-Chip, ist und/oder das optische Identifizierungselement (14) ein Strichcode (14) oder ein QR-Code ist und/oder das mechanische Identifizierungselement (15) Durchbohrungen (15), Wellenschliffe oder Kerben aufweist.

5. Röntgenröhre (9) mit einem Target (1) und/oder einem Filament (5) nach einem der vorstehenden Patentansprüche und mit einem Erfassungselement (13, 16, 18), das mit dem Identifizierungselement (8, 14, 15) des Targets (1) und/oder des Filaments (5) zusammenwirkt.

6. Röntgenröhre (9) nach Patentanspruch 5, wobei das Erfassungselement (13, 16, 18) eine elektronische Abtastvorrichtung, eine optische Kamera, eine Mechanik oder eine Sensorik ist.

7. Verfahren zur Erkennung eines konkreten Targets (1) gemäß einem der Patentansprüche 1, 3 oder 4 und/oder eines konkreten Filaments (5) gemäß einem der Patentansprüche 2 bis 4, wobei das an der Röntgenröhre (9) angebrachte Erfassungselement (13, 16, 18) das an der Targetbasis (10) und/oder am Filamenthalter angebrachte Identifizierungselement (8, 14, 15) erkennt und mittels einer Datenbank das Target (1) und/oder das Filament (5) eindeutig dem Identifizierungselement (8, 14, 15) zugeordnet wird.

8. Verfahren zur Erkennung eines konkreten Targets (1) wobei mittels einer Vergrößerung der Elektronenstrahlfläche auf dem Targetelement (11) bis auf die Außenfläche des Targetelements (11) aufgrund der targetspezifischen Brennfleckänderung eine eindeutige Zuordnung des Targets (1) erfolgt.

9. Verfahren zur Erkennung eines konkreten Targets (1), wobei beim Start der Röntgenröhre (9) zuerst eine immer gleiche vorgegebene Betriebsart angefahren wird, bei der bei gleichen Ansteuerparametern jedes Target (1) eine unterschiedliche Röntgenstrahlung emittiert, und die dabei entstehende Röntgenstrahlung ausgewertet wird.

10. Verfahren zur automatischen Einstellung der Kenngrößen eines in eine Röntgenröhre (9) eingebauten Targets (1) und/oder eines Filaments (5), wobei nach Durchführung eines Verfahrens gemäß einem der Patentansprüche 7 bis 9 die in der Datenbank für das jeweilige Identifizierungselement (8, 14, 15) beziehungsweise das Target (1) und/oder das Filament (5) abgelegten Kenngrößen an die Einstellvorrichtungen der jeweiligen Kenngrößen übertragen werden und von diesen Einstellvorrichtungen die Einstellung dieser Kenngrößen erfolgt.

## Claims

1. Target (1) for an X-ray tube (9) with a target base (10) and a target element (11) attached thereto, wherein there is mounted on the target base (10) an identification element (8, 14, 15) which can be identified in cooperation with an acquisition element (13, 16, 18) on the X-ray tube (9) and which has an unambiguous assignment to an appropriate set of parameters in the tube control system of the target (1).

2. Filament (5) for an X-ray tube (9) with a filament holder and a filament element attached thereto, wherein there is mounted on the filament holder an identification element which can be identified in cooperation with an acquisition element on the X-ray tube (9) and which has an unambiguous assignment to an appropriate set of parameters in the tube control system of the filament (5).

3. Target (1) or filament (5) according to one of the preceding claims, wherein the identification element (8, 14, 15) is an electrical identification element (8), an optical identification element (14) or a mechanical identification element (15).

4. Target (1) or filament (5) according to claim 3, wherein the electrical identification element (8) is an electronic component (8), in particular an RFID chip, and/or the optical identification element (14) is a barcode (14) or a QR code, and/or the mechanical identification element (15) has perforations (15), serrations or indentations.

5. X-ray tube (9) with a target (1) and/or a filament (5) according to one of the preceding claims and with an acquisition element (13, 16, 18) which cooperates with the identification element (8, 14, 15) of the target (1) and/or of the filament (5).

6. X-ray tube (9) according to claim 5, wherein the acquisition element (13, 16, 18) is an electronic scanning device, an optical camera, a mechanical system or a sensor system.

7. Method for identifying a specific target (1) according to one of claims 1, 3 or 4 and/or a specific filament (5) according to one of claims 2 to 4, wherein the acquisition element (13, 16, 18) mounted on the X-ray tube (9) identifies the identification element (8, 14, 15) mounted on the target base (10) and/or on the filament holder, and the target (1) and/or the filament (5) is unambiguously assigned to the identification element (8, 14, 15) by means of a database.

8. Method for identifying a specific target (1), wherein an unambiguous assignment of the target (1) takes place on the basis of the target-specific change in the focal spot by means of an enlargement of the electron beam area on the target element (11) up to the outer surface of the target element (11).

9. Method for identifying a specific target (1), wherein, when the X-ray tube (9) is started, first a predetermined operating mode which is always the same is started up in which, with the same control parameters, each target (1) emits a different X-radiation, and the resulting X-radiation is evaluated.

10. Method for automatically setting the characteristics of a target (1) and/or a filament (5) installed in an X-ray tube (9), wherein, after carrying out a method according to one of claims 7 to 9, the characteristics stored in the database for the respective identification element (8, 14, 15) or the target (1) and/or the filament (5) are transferred to the setting devices of the respective characteristics, and these characteristics are set by these setting devices.

## Revendications

1. Cible (1) pour un tube à rayons X (9) avec une base de cible (10) et un élément de cible (11) y étant fixé, un élément d'identification (8, 14, 15) étant placé au niveau de la base de cible (10), cet élément étant reconnaissance en interaction avec un élément de détection (13, 16, 18) au niveau du tube à rayons X (9) et comportant une association univoque à un jeu de paramètres adapté dans l'élément de commande de tube de la cible (1).

2. Filament (5) pour un tube à rayons X (9) avec un support de filament et un élément de filament y étant fixé, un élément d'identification étant placé au niveau du support de filament, cet élément étant reconnaissable en interaction avec un élément de détection au niveau du tube à rayons X (9) et comportant une association univoque à un jeu de paramètres adapté dans l'élément de commande de tube du filament (5).

3. Cible (1) ou filament (5) selon l'une quelconque des revendications précédentes, l'élément d'identification (8, 14, 15) étant un élément d'identification électrique (8), un élément d'identification optique (14) ou un élément d'identification mécanique (15).

4. Cible (1) ou filament (5) selon la revendication 3, l'élément d'identification électrique (8) étant un composant électronique (8), notamment une puce RFID et/ou l'élément d'identification optique (14) étant un code-barres (14) ou un code QR et/ou l'élément d'identification mécanique (15) comportant des alésages traversants (15), des couteaux dentelés ou des encoches.

5. Tube à rayons X (9) avec une cible (1) et/ou un filament (5) selon l'une quelconque des revendications précédentes et avec un élément de détection (13, 16, 18) interagissant avec l'élément d'identification (8, 14, 15) de la cible (1) et/ou du filament (5).

6. Tube à rayons X (9) selon la revendication 5, l'élément de détection (13, 16, 18) étant un élément de balayage électronique, une caméra optique, un système mécanique ou un système de détection par capteurs.

7. Procédé de détection d'une cible (1) concrète selon l'une quelconque des revendications 1, 3 ou 4 et/ou d'un filament (5) concret selon l'une quelconque des revendications 2 à 4, l'élément de détection (13, 16, 18) placé au niveau du tube à rayons X (9) reconnaissant des éléments d'identification (8, 14, 15) placés au niveau de la base de cible (10) et/ou du support de filament et la cible (1) et/ou le filament (5) étant associés de façon univoque à l'élément d'identification (8, 14, 15) au moyen d'une base de données.

8. Procédé de détection d'une cible (1) concrète, une association univoque de la cible (1) étant réalisée au moyen d'un agrandissement de la surface de faisceau d'électrons sur l'élément de cible (11) jusqu'à atteindre la surface extérieure de l'élément de cible (11) sur la base de la variation du point focal spécifique à la cible.

9. Procédé de détection d'une cible (1) concrète, sachant que lors du démarrage du tube à rayons X (9), c'est toujours le même mode de fonctionnement prédéfini qui est lancé pour lequel pour les mêmes paramètres de commande de chaque cible (1), un rayonnement aux rayons X différent est émis et analysé en l'occurrence en fonction du rayonnement aux rayons X produit.

10. Procédé de réglage automatique des caractéristiques d'une cible (1) et/ou d'un filament (5) encastrés dans un tube à rayons X (9), sachant qu'après la mise en oeuvre d'un procédé selon l'une quelconque des revendications 7 à 9, les caractéristiques placées dans la base de données pour l'élément d'identification (8, 14, 15) respectif et/ou la cible (1) et/ou le filament (5) sont transmises aux dispositifs de réglage des caractéristiques respectives et que le réglage de ces caractéristiques s'effectue à partir de ces dispositifs de réglage.
